# EUROPEAN PATENT APPLICATION

(11) **EP 0 563 434 A1**
(43) Date of publication of application: **06.10.1993**
(21) Application number: 92112470.7
(22) Date of filing: 21.07.1992
(51) Int. Cl.: C02F 3/28

(54) **Digester including sludge recirculator with gas supplement**

(30) Priority: 31.03.1992 US 861195
(71) Applicant: ENVIREX Inc., Waukesha Wisconsin 53186 (US)
(72) Inventor: Baumann, Peter Gene, Mukwonago, WI 53149 (US); Smith, George William, Pewaukee, WI 53072 (US)
(74) Representative: Grünecker, Kinkeldey, Stockmair & Schwanhäusser Anwaltssozietät

(57) **Abstract**

An anaerobic sludge digester (10) including a digester tank (1) and a sludge mixing system (26) for hydraulically mixing the contents of the digester tank and for agitating the surface of the sludge in the digester tank. The mixing system (26) includes a sludge supply assembly (28) and a gas supply assembly (48) for respectively supplying sludge and gas derived from the tank to a common distribution network (56) for discharge back into the tank. The distribution network includes a plurality of distribution pipes (72) and a sequencing mechanism (58,78) for simultaneously delivering a sludge and gas mixture to the pipes one at a time and in sequence.

## Description

### BACKGROUND OF THE INVENTION

### Field of the Invention

The invention relates to anaerobic sludge digesters, and more particularly to sludge digesters including systems for mixing the sludge within the digester tank to improve digestion and to prevent the unwanted accumulation of solid material in the digester.

### Reference to Prior Art

Anaerobic digestion systems are commonly used to bacteriologically treat waste such as sewage sludge before the byproducts of the treatment are discharged into the environment or otherwise disposed of. A typical digestion system includes a large digester tank for containing the sludge during digestion and piping for adding raw sludge to the tank and for removing partially digested sludge from the tank.

It is well known that to improve digestion, the digester contents must be periodically mixed or blended to reduce the settling and accumulation of solids in normally quiescent areas such as on the bottom of the digester tank. Mixing the sludge also distributes the bacteria to insure active digestion of the sludge, maintains uniform chemical and thermal conditions within the digester, and disperses toxic material entering the system to minimize its effect on the digestion process. A known digester mixing system for hydraulically mixing the contents of the digester tank is provided in U.S. Patent No. 4,092,338 issued May 30, 1978 to Tossey. This mixing system includes a network of pipes spread out over the base of the tank, each pipe leading to a separate discharge point in a different region of the digester tank. Sludge undergoing digestion is withdrawn from the digester tank and pumped through a slide valve which sequentially delivers the recirculated sludge to selected ones of the various pipes to mix sludge from less active regions of the digester tank into other regions.

It is also well known to introduce a gas into the digester tank to cause turbulence at the surface of the sludge to reduce the accumulation of scum on the surface. A known gas mixing system is produced by Envirex, Inc. of Waukesha, Wisconsin, and is known as a Pearth gas mixing system. In this system, methane gas collected from a gas dome on the cover of the digester tank is compressed and returned to the tank through a series of discharge lances. The discharge lances are spaced apart and extend downwardly from the cover into the sludge to a desired depth. A multi-port rotary valve between the compressor and the lances causes the gas to be sequentially discharged through the lances.

### SUMMARY OF THE INVENTION

The present invention provides a digester apparatus incorporating an improved mixing system in which sludge and gas streams are combined into a single stream before being discharged into the digester tank through a common distribution network. The distribution network includes a single sequencing mechanism through which the mixture of sludge and gas is sequentially delivered to only one discharge point at a time.

The prior art hydraulic mixing systems employed to mix sludge in anaerobic digesters are inadequate to provide sufficient turbulence at the surface of the sludge to avoid scum build-up. Redirecting some of the discharge points of the hydraulic mixing system to regions adjacent the sludge surface would not provide adequate coverage of the surface without reducing mixing benefits to other parts of the digester to unacceptable levels. Similarly, systems for agitating the surface of the sludge to reduce scum accumulation are inadequate to accomplish sufficient mixing throughout the digester tank. For these reasons, some prior art digesters have required the use of separate hydraulic and gas mixing systems, each mixing system including its own recirculation or distribution network and sequencing mechanism. The mixing system of the present invention eliminates the need for separate systems by achieving in a single, integrated system the benefits of both hydraulic mixing and gas mixing. This reduces the size, complexity and number of parts needed to accomplish successful mixing of the entire contents of the digester tank.

More specifically, the invention provides a sewage sludge digestion apparatus including a tank adapted to contain sewage sludge and wherein the sewage sludge is digested by bacteria, and a plurality of pipes within the tank. The pipes have respective effluent discharge ends which are spaced apart within the tank. The digestion apparatus also includes means for pumping partially digested sludge derived from the tank back into the tank to provide for mixing and agitation of the sludge contained in the tank to promote biological activity in the tank by the bacteria, and means for supplying partially digested sludge from the tank to the means for pumping. To distribute the partially digested sludge sequentially to at least one selected pipe at a time, valve means in communication with the means for pumping is provided. Means for supplying gas under pressure to the partially digested sludge being supplied to the valve means is also provided so that the partially digested sludge and gas are delivered together sequentially to the selected ones of the pipes.

The invention also provides a sewage sludge digestion apparatus including a tank adapted to contain sewage sludge and wherein the sewage sludge is digested by bacteria, a manifold including a plurality of effluent ports, and a plurality of pipes within the tank, each of the pipes communicating with the manifold through one of the effluent ports. Each of the pipes has an effluent discharge end, the effluent discharge ends of the pipes being spaced apart within the tank. Means are provided for supplying sludge to the manifold to distribute the sludge to the pipes for introduction into the tank. Means for supplying gas under pressure to the sludge being supplied to the manifold is also provided so that the gas under pressure is mixed with the sludge and travels together with the sludge through the pipes for introduction into the tank.

The invention further provides a sewage sludge digestion apparatus including a tank adapted to contain sewage sludge and wherein the sewage sludge is digested by bacteria, and a cover on the tank and including a gas collection reservoir. The digestion apparatus also includes a sludge supply assembly including means for pumping partially digested sludge derived from the tank back into the tank to provide for mixing and agitation of the sludge contained in the tank to promote biological activity in the tank by the bacteria. The sludge supply assembly also includes means for supplying partially digested sludge from the tank to the means for pumping, the means for supplying including a sludge recirculation conduit extending from the tank. To redistribute the sludge supplied by the sludge supply assembly, the digestion apparatus includes a distribution network including a plurality of pipes within the tank. Each of the pipes has a discharge end, the discharge ends of the pipes being spaced apart within the tank. The distribution network also includes valve means in communication with the means for pumping for distributing the partially digested sludge sequentially to at least one selected pipe at a time. The valve means includes a housing having an influent end portion and a manifold portion, the manifold portion including a plurality of effluent ports. Each of the pipes communicates with the manifold portion through one of the effluent ports. The valve means also includes a valve member housed in the housing for movement relative thereto to open and close selected ones of the effluent ports to facilitate the sequential distribution of the partially digested sludge to the selected ones of the pipes at a time. The distribution network also operates to deliver gas to the pipes to agitate the surface of the sludge in the tank. Thus, means are provided for supplying gas under pressure to the partially digested sludge being supplied to the valve means such that partially digested sludge and gas are delivered together sequentially to the selected ones of the pipes. The means for supplying gas under pressure includes a gas conduit communicating between the valve means and the gas collection reservoir and a compressor disposed within the gas conduit.

Other features and advantages of the invention are set forth in the following description, claims and drawings.

### BRIEF DESCRIPTION OF THE DRAWINGS

FIG. 1 is a partially diagrammatic side view, partially in section, of an anaerobic sludge digestion apparatus embodying various features of the invention.

FIG. 2 is a view taken along line 2-2 in FIG. 1, and shows in top elevation the distribution network of the digestion system.

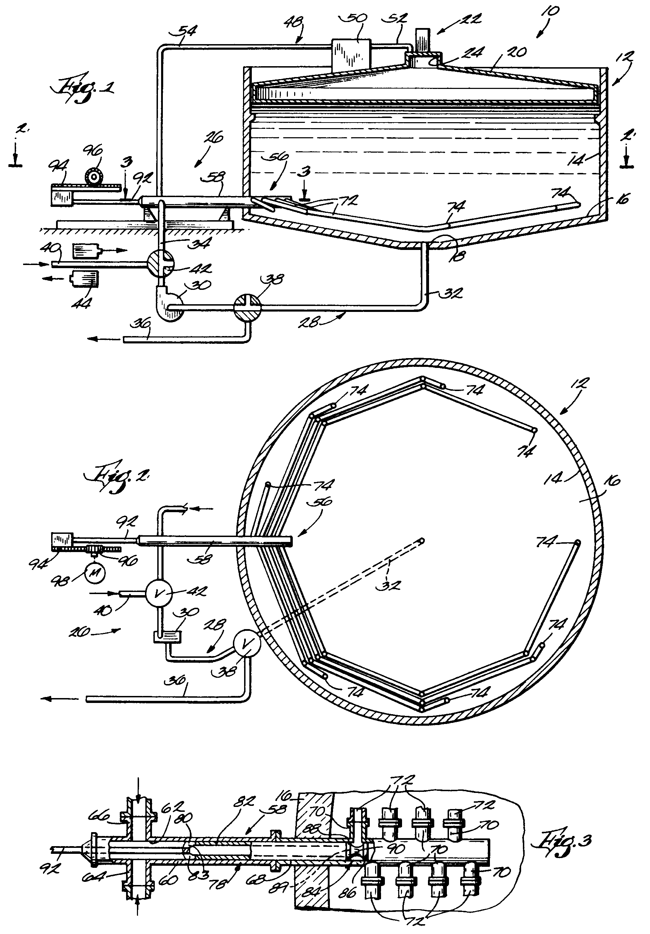

PIG. 3 is a view taken along line 3-3 in FIG. 1, and shows, partially in section, the mechanism for sequentially delivering a sludge and gas mixture to the various pipes of the distribution network.

Before one embodiment of the invention is explained in detail, it is to be understood that the invention is not limited to the details of construction and the arrangement of components set forth in the following description or illustrated in the drawings. The invention is capable of other embodiments and of being practiced or carried out in various ways. Also, it is to be understood that the phraseology and terminology used herein is for the purpose of description and should not be regarded as limiting.

### GENERAL DESCRIPTION

Illustrated in the drawings is an anaerobic sludge digestion apparatus 10 embodying various features of the invention. The digestion apparatus 10 forms part of a wastewater treatment facility and functions to contain organic solids or sewage sludge until the sludge is adequately digested by bacteria. Raw sludge is received in the digestion apparatus 10 after being separated from sewage water in another part of the treatment facility. The water is processed in other biological treatment steps and returned to the environment while the sludge is stabilized in the digestion apparatus 10 before also being returned to the environment or otherwise disposed of.

As shown in FIG. 1, the digestion apparatus 10 includes a digester tank 12 for containing the sludge while it is digested by the bacteria. The digester tank 12 includes a cylindrical wall 14 and a floor 16 which is generally conically shaped and which slopes downwardly toward a central sludge collection point or trough 18. The size of the digester tank 12 can be varied to suit the requirements of the installation, but is typically on the order of 100 feet in diameter and 30 feet high. The digester tank 12 is closed by a floating cover 20 including a centrally located gas dome 22 defining a gas collection reservoir 24 in which gases that evolve as a byproduct of the biological digestion process are collected. Such gases include methane and carbon dioxide, among others. Excess gas in the collection reservoir 24 can, if desired, be vented to the atmosphere or otherwise disposed of.

The digester apparatus 10 also includes a sludge distribution or mixing system for mixing or blending the sludge within the digester tank 12. As more fully explained below, the mixing system incorporates both a hydraulic mixing component for mixing the sludge in the digester tank 12 and a gas mixing component for agitating the surface of the sludge.

More specifically, the sludge mixing system 26 includes a sludge supply system or assembly 28 for supplying sludge to be introduced into the digester tank 12. In the illustrated arrangement, the sludge supply assembly 28 is operable to supply a mixture of raw sludge and recirculated partially digested sludge for introduction into the digester tank 12. However, it should be understood that in other constructions the sludge supply assembly 28 could be arranged to supply only recirculated sludge or only raw sludge for introduction into the digester tank 12.

As shown in FIGS. 1 and 2, the sludge supply assembly 28 includes a pump 30 for pumping partially digested sludge derived from the digester tank 12 back into the tank 12 to positively displace sludge in otherwise quiescent regions of the tank 12. The pump 30 is preferably a high volume, low head pump of conventional design, however, any suitable pumping means can be employed. A tank outlet pipe or suction conduit 32 extends between the bottom of the digester tank 12 and the pump 30. As shown in FIG. 1, the suction conduit 32 communicates with the collection trough 18 so that sludge which settles to the bottom of the digester tank 12 and slides down the floor 16 to the trough 18 is recirculated. A second sludge conduit 34 extends from the pump 30 for delivering the sludge pumped therefrom to a distribution system for delivering the sludge back into the digester tank 12, as will be further explained below.

The sludge supply assembly 28 also includes a discharge pipe or conduit 36 communicating with the suction conduit 32 through a valve 38. The valve 38 regulates the flow of partially digested sludge entering the discharge conduit 36 from the suction conduit 32 for ultimate disposal at a remote location. While in the illustrated arrangement partially digested sludge can be withdrawn from the sludge supply assembly 28 for disposal, in other arrangements this sludge can be withdrawn from the digester apparatus 10 at other locations or using other suitable means.

The sludge supply assembly 28 is also provided with a sludge inflow pipe or conduit 40 for supplying raw sludge to the digester apparatus 10. A second valve 42 regulates the flow of raw sludge into the sludge conduit 34. While in the illustrated arrangement raw sludge is introduced into the partially digested sludge being pumped from the digester tank 12, in other arrangements raw sludge can be introduced at other locations in the system using other suitable means.

To maintain desired thermal conditions within the digester tank 12, a heat exchanger 44 is provided to heat the raw sludge flowing through the inflow conduit 40 before its introduction into the sludge conduit 34. It should be understood that one or more heat exchangers could be employed at other locations to heat either or both the raw sludge and the recirculated sludge, if desired.

The sludge mixing system 26 also includes a gas supply system or assembly 48 for supplying gas to be introduced into the digester tank 12 so that the gas bubbles to the surface of the sludge to create surface turbulence. This surface agitation is desired to prevent the accumulation of scum on the surface of the sludge.

The gas supply assembly 48 includes means for supplying gas under pressure to the partially digested sludge (and raw sludge if present) so that the sludge and the gas form a mixture before being introduced into the digester tank 12. While various pressurized gas supply means can be used, in the illustrated arrangement such means recycles gas collecting in the gas dome 22 and includes a compressor 50 communicating with the gas collection reservoir 24 through a gas recirculation pipe or conduit 52. While gas from the gas dome 22 is preferably recycled, any gas source capable of supplying suitable gas that does not contain amounts of oxygen sufficient to interfere with anaerobic digestion could be used. The compressor 50 is located on the floating cover 20, but could also be located elsewhere such as in a nearby building (not shown) or other suitable remote location. A second gas pipe or conduit 54 extends from the compressor 50 for delivering the pressurized recycled gas to the aforementioned distribution system for its ultimate reintroduction into the digester tank 12, as explained below.

The mixing system 26 also includes a distribution system or network 56 which receives the recirculated sludge and gas from the sludge and gas supply assemblies 28 and 48 and distributes this mixture back into the digester tank 12. As shown in FIG. 3, the distribution network 56 includes a housing 58 having a tubular influent end portion 60 defining an entry chamber 62. The influent end portion 60 is provided with a pair of elbows 64 and 66 respectively connected to the sludge conduit 34 and the gas conduit 54 so that recirculated sludge and gas are introduced into the entry chamber 60 where they are mixed. The housing 58 also includes a tubular manifold portion 68 connected to the influent end portion 60 and extending into the digester tank 12. The manifold portion 68 includes a plurality of effluent ports 70 arranged in spaced apart, staggered relation along the length of the manifold portion 68.

To deliver the recirculated sludge and gas mixture from the housing 58 to the digester tank 12, the distribution network 56 includes a plurality of distribution pipes 72 each connected to one of the effluent ports 70. The pipes 72 are positioned above and closely adjacent the floor 16 of the digester tank 12 and each includes a discharge end 74. It is preferred that the discharge ends 74 of the pipes 72 be oriented to sweep sludge from different regions of the floor area in which sedimentation would otherwise be expected to occur. Thus, the pipes 72 are of various lengths and some are appropriately bent or jointed to generally follow the contour of the cylindrical wall 14 of the digester tank 12 so that the discharge ends 74 of the pipes 72 are spaced around the tank 12 and along the cylindrical wall 14. The number and arrangement of the pipes 72 in the distribution network 56 can be varied from what is shown in the drawings. For example, the pipes 72 could be arranged in a fan-shaped configuration such as is disclosed in Patent No. 4,092,338 which is herein incorporated by reference.

Due to the high power requirements that would be needed to forcefully deliver sludge and gas to all of the pipes 72 at the same time, it is preferred that delivery occur to only selected ones of the pipes 72 at a time. To sequence the delivery of the sludge and gas mixture to the various discharge points served by the pipes 72, the distribution network 56 includes a valve means for selectively distributing the mixture to one of the pipes 72 at a time. While various valve means can be employed, in the illustrated arrangement the valve means is of the type disclosed in Patent No. 4,092,338. Thus, the valve means incorporates the tubular housing 58 and includes a sliding valve apparatus 78 reciprocally movable within the housing 58.

The valve apparatus 78 includes a vane member 80 attached to the proximal end of a hollow tube 82, the vane member including openings 83 communicating with the interior of the tube 82. The valve apparatus 78 also includes a valve member 84 which is attached to the distal end of the tube 82 and which travels within the manifold portion 68. The valve member 84 includes a circular seal member 86 which slideably seals to the interior wall of the manifold portion 68. The valve member 84 also includes a ring 88 which has holes or openings 89 communicating with the interior of the tube 82, and a central web 90 interconnecting the ring 88 and the seal member 86. When a selected one of the effluent ports 70 is centered between the seal member 86 and the ring 88, the sludge and gas mixture flows from the entry chamber 62 through the openings 83 in the vane member 80, the tube 82, and the holes 89 in the ring 88 to the distribution pipe 72 corresponding to the selected port 70.

To advance the valve apparatus 78, the valve means includes an operating rod 92 connected to the vane member 80 and extending outside the housing 58 to a rack 94. The rack 94 is drivingly engaged by a pinion 96 which is driven by a motor 98. Indexing circuitry or a manual switch (not shown) can be used in conjunction with the motor 98 to advance the valve apparatus 78 so as to sequentially direct the recirculated material from the entry chamber 62 to the distribution pipes 72.

The distribution network 56 can be employed as a retrofit device in digester apparatus which were originally equipped with other sludge and/or gas mixing systems to replace or supplement these systems. Where the distribution apparatus 56 is built into a newly constructed digester apparatus, the pipes 72 can be partially embedded in the concrete which forms the tank 12.

Operation of the mixing system 26 to circulate the sludge within the digester tank 12 and to prevent the build-up of scum on the surface of the sludge is as follows. Partially digested sludge and gas from the digester tank 12 are simultaneously pumped through the sludge supply assembly 28 and gas supply assembly 48, respectively, to the entry chamber 62 of the distribution network 56. The sludge and gas mixture traveling through the distribution network 58 is sequentially delivered, via operation of the valve member 84, to the pipes 72. As the mixture is forcefully emitted from the discharge end 74 of a selected one of the pipes 72, the sludge in the region of the digester tank 12 served by that pipe 72 is hydraulically mixed to provide mixing turnover in that region. This prevents settleable solids from accumulating in significant amounts on the portion of the floor 16 in the region and insures adequate digestion in the region. By sequentially discharging the sludge and gas mixture through all of the pipes 72 the entire contents of the digester tank 12 can be hydraulically mixed. While the gas emitted through the pipes 72 may provide some hydraulic mixing benefits, the primary mission of the gas is to rise to the sludge surface to agitate the surface, thereby preventing the accumulation of scum thereon. The positioning of the discharge ends 74 of the pipes 72 around the tank 12 facilitates substantially complete coverage of the surface.

Advantageously, the mixing system 26 provides both hydraulic mixing for circulating the sludge within the tank to insure active digestion throughout substantially the entire digester tank volume, and gas mixing for agitating the surface of the sludge to avoid scum build-up. By combining previously separate hydraulic and gas mixing systems into a single system, a single distribution network can be used to introduce both the recirculated gas and sludge into the digester tank 12. Since only a single distribution network 56 is employed, only one sequencing mechanism, i.e. the valve apparatus 78, is required to simultaneously sequence the delivery of sludge and gas to the various discharge points. Accordingly, the digester apparatus 10, and particularly the mixing system 26, includes fewer parts and is less expensive to operate and maintain than are prior art arrangements employing separate hydraulic and gas mixing systems.

Various features of the invention are set forth in the following claims.

## Claims

1. A sewage sludge digestion apparatus comprising:
a tank adapted to contain sewage sludge and wherein the sewage sludge is digested by bacteria;
a plurality of pipes within the tank, each of the pipes having an effluent discharge end, the effluent discharge ends of the pipes being spaced apart within the tank;
means for pumping partially digested sludge derived from the tank back into the tank to provide for mixing and agitation of the sludge contained in the tank to promote biological activity in the tank by the bacteria;
means for supplying partially digested sludge from the tank to the means for pumping;
valve means in communication with the means for pumping for distributing the partially digested sludge sequentially to at least one selected pipe at a time; and
means for supplying gas under pressure to the partially digested sludge being supplied to the valve means such that partially digested sludge and gas are delivered sequentially to the selected ones of the pipes.

2. A sewage sludge digestion apparatus as set forth in claim 1 wherein the valve means includes a housing including an influent end portion and a manifold portion having a plurality of effluent ports, each effluent port communicating with one of the pipes, and wherein each of the means for pumping partially digested sludge and the means for supplying gas under pressure communicates with the influent end portion of the valve means such that the partially digested sludge and the gas under pressure mix within the valve means.

3. A sewage sludge digestion apparatus as set forth in claim 2 wherein the valve means includes a valve member housed in the housing for reciprocal movement relative thereto to selectively open and close the effluent ports to facilitate the sequential distribution of the mixture of partially digested sludge and gas under pressure to one of the pipes at a time.

4. A sewage sludge digestion apparatus as set forth in claim 1 wherein the means for supplying gas under pressure includes means for pumping gas produced within the tank to the valve means.

5. A sewage sludge digestion apparatus as set forth in claim 1 wherein the sludge digestion apparatus includes a floating cover on the tank and including a gas collection dome, and wherein the means for supplying gas under pressure includes a gas conduit communicating between the valve means and the gas dome and a compressor disposed within the gas conduit.

6. A sewage sludge digestion apparatus comprising:
a tank adapted to contain sewage sludge and wherein the sewage sludge is digested by bacteria;
a manifold including a plurality of effluent ports;
a plurality of pipes within the tank, each of the pipes communicating with the manifold through one of the effluent ports, and each of the pipes having a discharge end, the discharge ends of the pipes being spaced apart within the tank;
means for supplying sludge to the manifold to distribute the sludge to the pipes for introduction into the tank; and
means for supplying gas under pressure to the sludge being supplied to the manifold so that the gas under pressure is mixed with the sludge and travels with the sludge through the pipes for introduction into the tank.

7. A sewage sludge digestion apparatus as set forth in claim 6 wherein the sludge digestion apparatus includes a valve assembly associated with the manifold for closing selected effluent ports of the manifold to distribute the mixture of sludge and gas under pressure sequentially to at least one selected pipe at a time.

8. A sewage sludge digestion apparatus as set forth in claim 7 wherein the means for supplying sludge to the manifold includes means for pumping partially digested sludge derived from the tank back into the tank to provide for mixing and agitation of the sludge contained in the tank to promote biological activity in the tank by the bacteria, and means including a sludge recirculation conduit for supplying partially digested sludge from the tank to the means for pumping.

9. A sewage sludge digestion apparatus as set forth in claim 8 wherein the sludge digestion apparatus includes a floating cover on the tank and including a gas collection reservoir, and wherein the means for supplying gas under pressure includes a gas conduit communicating between the manifold and the gas collection reservoir and a compressor disposed within the gas conduit.

10. A sewage sludge digestion apparatus comprising:
a tank adapted to contain sewage sludge and wherein the sewage sludge is digested by bacteria;
a cover on the tank and including a gas collection reservoir;
a plurality of pipes within the tank, each of the pipes having a discharge end, the discharge ends of the pipes being spaced apart within the tank;
means for pumping partially digested sludge derived from the tank back into the tank to provide for mixing and agitation of the sludge contained in the tank to promote biological activity in the tank by the bacteria;
means for supplying partially digested sludge from the tank to the means for pumping, the means for supplying including a sludge recirculation conduit extending from the tank;
valve means in communication with the means for pumping for distributing the partially digested sludge sequentially to at least one selected pipe at a time, the valve means including a housing having an influent end portion and a manifold portion, the manifold portion including a plurality of effluent ports, each of the pipes communicating with the manifold portion through one of the effluent ports, and the valve means including a valve member housed in the housing for movement relative thereto to open and close selected ones of the effluent ports to facilitate the sequential distribution of the partially digested sludge to selected ones of the pipes at a time, wherein the means for pumping partially digested sludge communicates with the influent end portion of the valve means; and
means for supplying gas under pressure to the partially digested sludge being supplied to the valve means such that partially digested sludge and gas are delivered sequentially to the selected ones of the pipes, the means for supplying gas under pressure including a gas conduit communicating between the influent end portion of the valve means and the gas dome and a compressor disposed within the gas conduit so that the gas under pressure is delivered to the valve means such that the partially digested sludge and the gas under pressure mix within the valve means.
